# EUROPEAN PATENT SPECIFICATION

(11) **EP 0 502 107 B1**
(45) Date of publication and mention of the grant of the patent: **22.01.1997**
(21) Application number: 91900570.2
(22) Date of filing: 13.11.1990
(51) Int. Cl.: C12N 7/08, C07K 16/00, C07K 16/08, A61K 39/00, A61K 38/00, A61K 35/76, G01N 33/536, G01N 33/541, G01N 33/567

(54) **TYPE-B ROTAVIRUS CULTURES AND USES THEREOF**
ROTAVIRUS-TYP-B-KULTUREN UND DEREN VERWENDUNG
CULTURES DE ROTAVIRUS DU TYPE B ET APPLICATIONS DE CELLES-CI

(30) Priority: 13.11.1989 US 434209
(43) Date of publication of application: 09.09.1992
(73) Proprietor: AMBICO INC., Dallas Center, IA 50063 (US)
(72) Inventor: WELTER, Mark, W., Urbandale, IA 50322 (US); CHAMBERS, David, M., Des Moines, IA 50310 (US); WELTER, C., Joseph, Des Moines, IA 50321 (US)
(74) Representative: Andersen, Henrik Rastrup
(86) International application number: US9006622
(87) International publication number: WO9107482

(56) References cited:
- Journal of Virology, Volume 63, No. 5, Issued May 1989, Fang et al, pages 2191-2197
- Journal of Clinical Microbiology, Volume 27, No. 2, Issued February 1989, Burns et al, pages 245-798.
- Journal of Clinical Microbiology, Volume 27, No. 4, Issued April 1989, Mattion et al, pages 795-798.

## Description

### Technical Field

The field of this invention relates to propagation of Type-B rotaviruses in cell culture and subsequent production of antigen and antiserum for use in diagnostic kits and production of vaccines (Modified Live and Killed) to prevent Type-B rotavirus infections.

### Information Disclosure Statement

Rotavirus is the leading cause of viral gastroenteritis in infants and piglets (2,3,4,6,10,13,25). Rotaviruses, which are found in a great variety of animal species, are named for their characteristic wheel-like appearance under the electron microscope. Like other Reoviridae, their genome is in the form of double-stranded (ds) RNA, but they may be distinguished from reoviruses and orbiviruses by the division of their genome into 11 dsRNA segments.

In 1983, Pedley (31) classified the rotaviruses into several types or groups on the basis of serological differences, determined by immunofluorescence, and nucleic acid differences, characterized by one dimensional terminal fingerprint analysis. RNA electropherotype has also been used as a basis for classification (17). Group A rotaviruses are considered "typical"; all others (B, C, D, E) are referred to as "atypical." Group B rotaviruses are of particular interest because of their ability to infect a wide range of animals and to cause human disease. A monoclonal antibody against a group reactive-epitope of Group B rotaviruses has been developed and may be used in an immunoassay for viral characterization (26).

Burns et al. (Journal of Clinical Microbiology, Vol. 27, No. 2, 1989) disclose several monoclonal antibodies which react with natural isolates of human group B rotavirus.

Type B (also known as Group B) rotavirus has been implicated in diarrhea of nursing and weanling pigs (10,20). Diagnostic surveys conducted over several years have revealed that Type-B rotavirus infections in pigs are responsible for 25% of the rotavirus positive cases (10). Serological surveys have shown Type-B rotavirus antibody in 23 % of the swine sera tested in an Ohio State study and 86% of sera from five different herds in the United Kingdom (5,19). To date, Type-B rotavirus has been isolated from humans, cattle, rats, swine, sheep and chickens, but has not been successfully propagated in cell culture (1,7,13,16,17,20,21,22). Type-B rotaviruses from different sources are believed to have a common group antigen. Type B rotavirus antiserum has been demonstrated to cross-react with human (ADRV), rat, bovine, swine and lamb Type-B rotaviruses (6,17,26). Some evidence actually suggests that Type-B rotavirus isolates may be virulent for species of animals other than that of the animal from which they were isolated (8,26).

Type-A rotaviruses have been successfully propagated in several different cell lines, but require incorporation of either proteolytic enzymes, DEAE dextran or a combination of both. There are two U.S.D.A. licensed, modified live (MLV) Type-A rotavirus vaccines. The first vaccine was developed by Norden Laboratories, a MLV rotavirus vaccine for use in cattle. The bovine rotavirus vaccine consists of one strain of Type-A bovine rotavirus (NCDV) (27-29). The second vaccine was developed by Ambico Inc. for use in swine and consists of the two major serotypes of Type-A porcine rotavirus. The Ambico Porcine Rotavirus vaccine is also a MLV vaccine and safety and efficacy have been reported on previously (11,12,23,24). These vaccines are used as a means of controlling rotavirus diarrhea in animals. Successful control of rotavirus infections in animals must include both passive and active immunity. The vaccines may be used in both the dam (passive immunity) and the nursing animal (active immunity). Human vaccines containing rotavirus Type-A have also been developed but not yet commercialized (14,30). Diagnostic reagents have also been commercially available for diagnosis of Type-A rotavirus infections.

Successful propagation of rotavirus Type B has not been previously reported. Nakata (15) reports an unsuccessful attempt to apply Group A passaging techniques to Group B rotaviruses. Rotavirus Type C has only been maintained, with difficulty, a limited number of times in cell cultures (16). Although all rotavirus types appear morphologically similar, each type has their own antigen components which do not cross-react between types. There is an obvious need for the development of diagnostic aids and vaccines for rotavirus Type B in animals and humans. A process for propagating rotavirus Type B in cell culture would facilitate the development of these desirable products.

Fang et al. (Journal of Virology, Vol. 63, No. 5, 1989) have demonstrated that a 47 kDa inner capsid polypeptide derived from group B rotaviruses is immunogenic, but not that the immune response to it has a protective effect. Furthermore, Fang et al. concede that "work with human isolates of nongroup A rotaviruses has been hampered by an inability to grow these viruses in cell culture" (page 2191, column 1, last four lines). Fang's isolate is not attenuated; since the art did not know how to passage rotavirus B, by definition it could not attenuate it.

### SUMMARY OF THE INVENTION

This invention is based upon a method of treating fluids containing Type-B rotavirus to activate the virus, thus allowing for its growth and maintenance in cell cultures. To adapt the virus to cell culture, it must be treated with a chelating agent such as EDTA. Type A and C rotavirus growth is characterized by their dependence on concentrations of proteolytic enzymes to enhance or even allow for virus replication in cell culture. Attempts to use proteolytic enzymes in conjunction with and without EDTA in growth of Type-B rotavirus resulted in drastic loss of virus titer. The use of EDTA in Type-A and C rotavirus growth has been found to make the virus noninfectious by removing the outer shell (9), thus, this procedure is unique to Type-B rotavirus isolates and is the only method at this time that allows for consistent growth and continued propagation in cell cultures without changing the viral genome.

Type B rotavirus growth is characterized by the production of a unique cytopathic effect (CPE) in the infected cell cultures unlike that described for cell culture adapted strains of Type A rotaviruses. Infected cell cultures demonstrate syncytial-like focal areas of infection and are confirmed as Type-B rotavirus by specific Indirect Immunofluorescent (IFA) staining of cultures or by RNA extraction and polyacrylamide gel electrophoresis (PAGE) evaluations of the harvested fluids. It is therefore, an object of the present invention to serially propagate the Type B rotavirus in cell cultures in such a manner that its unique CPE is observable. This rotavirus may be passaged in the same or in a variety of cells sufficiently to attenuate its pathogenicity, whereby the attenuated virus may, in a suitable carrier, serve as a modified live virus vaccine. The propagated virus is also a source of viral antigens for therapeutic (including prophylactic) and diagnostic use.

Pigs inoculated with cell culture passaged Type-B rotavirus respond immunologically and produce anti Type-B rotavirus antibodies as measured by IFA, serum neutralizing and ELISA assays. Several strains of Type-B rotavirus have been propagated using this method, including four genomic-different Type B porcine rotavirus isolates and the human Type-B rotavirus (ADRV str) isolate originating from China. Serum to the human Type-B rotavirus (ADRV) and to the porcine Type-B rotavirus have been found to cross react (IFA) with each other suggesting a relationship between the two strains of Type-B rotavirus. In addition hyperimmune serum to Human Type-B rotavirus has been reported to cross react with the Type-B bovine rotavirus, Type-B lamb rotavirus and Type-B rat rotavirus (6,15,26). It is therefore apparent in the examples presented herein that the cell culture adapted porcine type-B rotavirus be used as a substitute for human Type-B rotavirus, for various purposes. Thus, it can serve as an indicator virus for production of antiserum and as an antigen source for use in diagnostic kits. In addition the cell culture adapted porcine Type-B rotavirus described herein is also a viable vaccine candidate for Type-B rotavirus infections in porcine, human and other species.

It is a further object of the present invention to provide rotavirus Type B antigen for use in diagnostic tests suitable for all animal (and human) species. It is yet another object of the present invention to provide a vaccine against porcine Type B rotaviruses, said vaccine also serving as an immunogen for protecting other nonporcine animals and humans against rotavirus Type B disease.

Other objectives and advantages will become apparent from the description of the present invention.

### BRIEF DESCRIPTION OF DRAWINGS AND FIGURES

These and other objects, features and many of the attendant advantages of the invention will be better understood upon a reading of the following detailed description when considered in connection with the accompanying drawings herein:

FIG. 1: Shows the virus genomes ("fingerprint") of a Porcine Type-B rotavirus isolate by means of the RNA extraction and PAGE analysis technique (18). Both the Virulent (Intestinal origin Parent) and cell culture adapted (passes 25 and 50) isolates showed the same fingerprint. Cell culture passages utilized Ma-104 cells.

FIG. 2: Shows the virus genomes of 4 different Porcine Type-B cell culture adapted rotaviruses. Each strain of rotavirus was passaged 10 times in Ma-104 cell cultures.

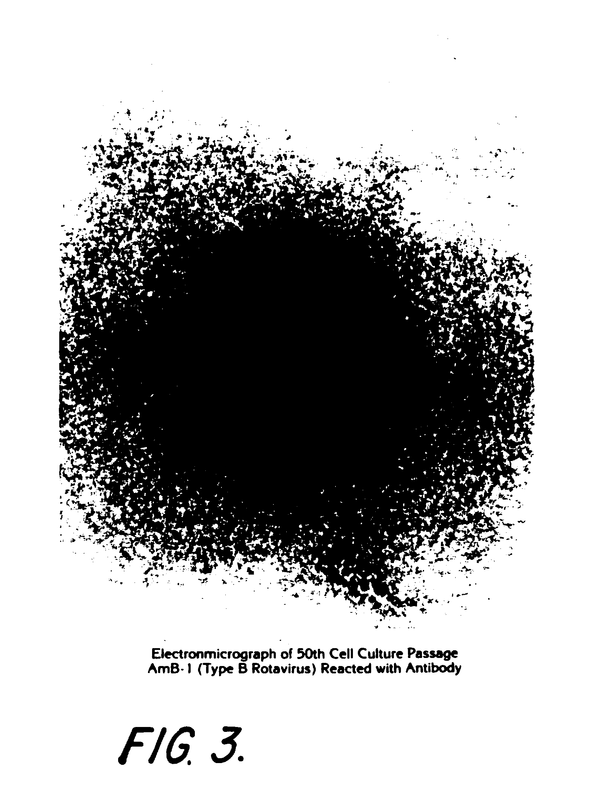

FIG 3: Immune electron micrograph (IEM) of the 50th pass of the porcine Type-B rotavirus in Ma-104 cells demonstrating the virus to have maintained its antigenicity through cell culture passage.

### DETAILED DESCRIPTION OF THE PREFERRED EMBODIMENTS

The above objectives and advantages of the present invention are achieved as illustrated by the examples set forth herein.

Heretofore, a rotavirus Type B has not been successfully grown and passaged in cell culture. The cell culture adapted Type-B porcine rotavirus provides a distinct advantage over human strains due to its lack of pathogenicity. Results of indirect immunofluorescent staining of infected cell cultures clearly establish the antigenic relationship between the two strains.

For the practice of the present invention, any materials and methods equivalent to those described herein can be used, but the more preferred choices are presented hereunder.

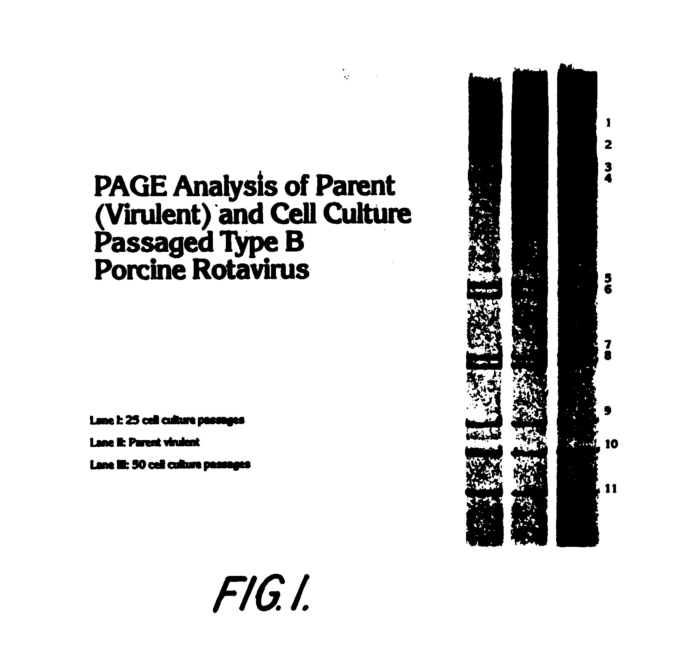

The isolate herein designated Ambico Type-B strain-1 (AmB-1) was isolated from an infected herd in Iowa. The Type-B virus was isolated from a small intestinal extract of an infected five week old, recently weaned diarrheic pig. This isolate was purified by centrifugation and filter sterilization and inoculated orally into 30 day old gnotobiotic pigs which had previously been orally exposed to two serotypes of Type-A porcine rotavirus. The pigs had recovered from the Type-A rotavirus exposure before oral inoculation with the AmB-1 rotavirus. The animals were sacrificed at onset of diarrhea (2-days post-inoculation) and a new intestinal extract was prepared using the entire intestinal contents. The virus intestinal contents were passed in gnotobiotic pigs two more times (incubating with anti Type-A and anti Type-C rotavirus serum prior to inoculation). Only type-B rotavirus was identified by the fourth passage in gnotobiotic pigs. Its purity and identity was demonstrated by: (a) Specific Indirect IFA staining of small intestinal sections of challenged sacrificed animals(18 hours post-challenge); (b) Viral Genome profile by RNA extraction PAGE evaluation of intestinal contents (Figure 1) and (c) Immunelectron microscopy of intestinal contents. Purity was further demonstrated as the intestinal contents were used to produce hyperimmune serum in colostrum deprived Cesarean derived (CDCD) pigs and only activity to Type-B rotavirus was detected in the serums post hyperimmunization.

Virulence of the Type-B bulk virus was demonstrated in Cesarean derived colostrum deprived (CDCD) pigs. Challenged pigs developed watery diarrhea within 24 hours post-inoculation and it persisted for 5 to 7 days post-challenge. Small intestinal segments taken from pigs sacrificed at 18-hours post-challenge, revealed a fused and stunted appearance of the small intestinal enterocytes (villous atrophy). Frozen sections stained by IFA revealed focal areas of infection (syncytia-like) with the predominate infection occurring in the duodenum and jejunum of the intestine. The lumen of small intestine was full of watery contents and challenged animals demonstrated a decreased average daily weight gain compared to nonchallenged controls. The pig infectious dose (PID) was determined to be 10³ PID₅₀/ml with a duration of 5 to 7 days.

The bulk intestinal fluids described earlier were used as starting material for growth in cell culture. With the use of techniques and media described below the following bovine, porcine and monkey cell lines were found to support growth of the type-B rotavirus: BT (Bovine turbinate) and Embryonic Bovine Kidney (EBK); ST (Swine Testicular) and PK (Primary Pig Kidney) and Vero (African Green), BSC (African Green) CV-1 (African Green Monkey Kidney), EBL (Embryonic Bovine Lung), and Ma-104 (Embryonic Rhesus Kidney). The virus could be propagated in both suspension and monolayer cultures. Other cell lines besides those mentioned here may be used. The cell line of choice was Ma-104 and all following virus propagation data was obtained in Ma-104. The Ma-104 cells were obtained from Dr. Ed. Bohl at Ohio State Agricultural Research and Development. Ma-104 cells are also available from Dr. Prem Paul (Iowa State U.) or Dr. Harry Greenberg (Stanford U.). These cells are widely used in many labs. After the Ma-104 cell line, the best hosts were the EBK and ST cells.

In adapting the Type-B rotavirus to cell culture it was discovered that the virus had to be treated with a chelating agent, preferably one which chelates Ca++ and Mg++ ions and, more preferably, an amino carboxylic acid (e.g., EDTA, EGTA, DTPA or benzyl-EDTA). Typically, the concentration of the chelating agent is 0.1mM-100mM, with 1-10mM being preferred. The virus may be pretreated with the chelating agents, or the cells may be treated after virus adsorption, with pretreatment being the more effective technique. The effect of the chelating agent on the virus is unclear at this time, but studies in which virus propagation medium was further supplemented with calcium or magnesium ions demonstrated a decrease in the virus infectivity for cell culture. This might suggest that a medium deficient in or with low concentrations of calcium or magnesium may be as effective as chelating agent treatments. It is also possible that the chelating agent activates the viral polymerase, in which case the invention embraces other chelating agents having such effect. Yet another alternative is the use of a detergent, such as SDS or Tween-20, to activate the virus. Note that calcium and magnesium ions are chelated by EDTA and EGTA. EGTA and EDTA appear to be equally effective.

Once the Rotavirus B has been adapted to cell culture with the aid of the chelating agent, it may be weaned from the chelating agent. Such weaning may be abrupt, or by progressive reduction in chelating agent concentration. When weaning is abrupt, it is preferably no earlier than after 10 passages, and more preferably after 20 passages. After weaning, it continues to produce the characteristic cytopathic effect and group activity.

Mixed serotype rotavirus infections are common in the field (10). By treating the virulent sample with chelating agent for growth of the Type-B rotavirus and passaging the virus it is possible to indirectly purify the Type-B virus from Types A and C as chelating agent will render the latter rotaviruses noninfectious.

Several strains of Type-B rotavirus have been successfully cultivated in cell culture by the method of the present invention.

Vaccines are prepared using an effective amount of the Type-B rotavirus prepared according to the present invention and combined with a pharmaceutically acceptable carrier. Depending upon the type of administration proposed for the vaccine, the carrier may be one suitable for oral, intramuscular, or other conventional type of vaccine administration. For a modified live virus vaccine, the virus is preferably lyophilized and stabilized with sucrose, gelatine and peptone. For a killed virus (or antigenic fraction) vaccine, preferred carriers are Freund's complete or incomplete adjuvants, squalane, and aqueous aluminum hydroide. Moreover, the Type-B rotavirus produced by the present invention may be incorporated in any conventional multivalent vaccine formulations including with Type-A rotavirus, transmissible gastroenteritis, Clostridium perfringens Type-C, and Escherichia coli, either alone or in any suitable combination thereof.

The Type-B rotavirus antigen produced according to the present invention can be used in diagnostic tests to diagnose infections with Type-B rotavirus. These types of immunological tests are well known to those skilled in the art, and include RIA, ELISA, immunofluorescence, immunoagglutination, and the like.

Crude or purified Type B rotavirus antigens may also be used as immunogenic agents in the production of antibody-containing fluids or in the vaccination of susceptible subjects against Type B rotavirus, or as immunosorbents in the purification of Type B rotavirus-specifc antibodies. These polyclonal or monoclonal antibodies, in turn, may be used therapeutically as a substitute for or in addition to induction of an immune response by vaccination. The antibodies may be administered in the form of immune serum or milk, or in a more purified form. The antibodies may also be labeled or insolubilized for use as diagnostic agents.

While Type-B rotavirus particles may be obtained from the culture media used to support the growth of the host cells, yields are improved if the host cells are lysed, e.g., by several freeze-thaw cycles or by sonication. The viral fluids are then purified from the cellular debris, e.g., by centrifugation at 2,000 to 6,000 xg. Optionally, the virus may be further purified, e.g., by ultracentrifugation at 100,000 xg through a 20% sucrose cushion and resuspended in physiological saline. The resulting antigenic preparation may then be labeled or immobilized for diagnostic use.

Alternatively, the preparation may be chromatographically resolved by, e.g., gel filtration, ion exchange chromatography, lectin affinity chromatography, reverse phase HPLC, etc. into component antigenic fractions or molecules.

### EXAMPLE 1

### VIRUS PROPAGATION AND SERIAL PASSAGE IN Ma-104 CELL LINE

Medium used for virus propagation consists of Eagle's minimal essential medium with nonessential amino acids and L-glutamine in Earles BSS, 0.1 M Sodium pyruvate, pH adjusted to 7.2 with NaHCO₃. Just prior to use the basal medium is further supplemented with 0.2 M Hepes buffer, 10 mM 1-glutamine, 0.05 µg/ml DEAE Dextran, 50 µg/ml gentamicin, and pH adjusted to 7.0 to 7.2 with 10 N NaOH. The use of DEAE-dextran during viral adsorption was found to increase the number of focal areas and thereby viral infectivity. Serum (2%) was also found to be beneficial in this manner, though its presence hinders propagation of Type A rotaviruses. L-glutamine and pyruvate likewise increased the number of foci. Cell cultures are rinsed 1-3x and incubated at 37°C for 30 to 60 minutes prior to inoculation. Cell culture vessels that were used for virus propagation include; 48 well microtiter plates, tissue culture tubes, leighton tubes, 32 oz bottles and 640 cm² roller bottles. Rolling was found to be the most effective method of generating virus but was not a necessity as virus growth was also achieved in stationary cultures.

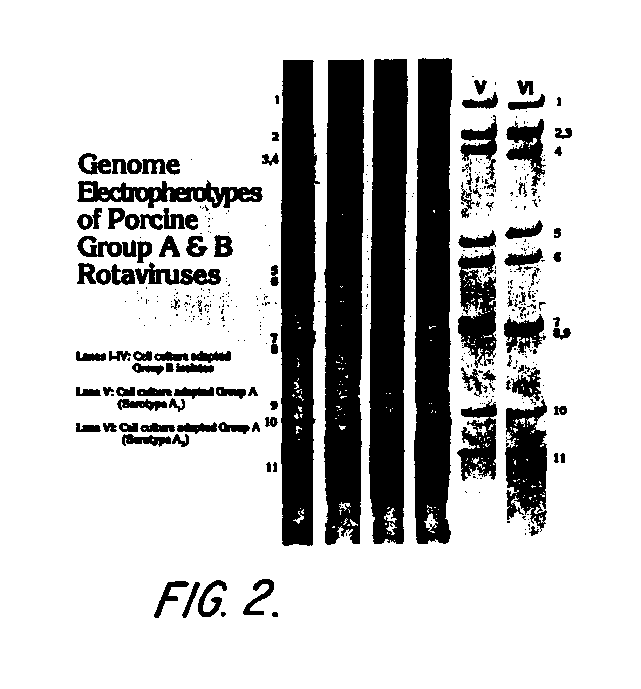

Virus fluids for inoculation are pretreated with a 1 to 10 mM concentration of a chelating agent (e.g., EDTA or EGTA) for 5 to 15 minutes at 37°C. The solution can be diluted prior to inoculation of cells. The rinse medium is removed and the cultures are inoculated with the pretreated Type-B rotavirus and incubated at 37-39°C for 1-2 hours. The inoculum is rinsed off and fresh virus propagation medium added. The cultures (either rolled or stationary) are incubated at 37-39°C for 1-7 days. A unique CPE is observed as early as 18 hours post-inoculation (Figure 2). Infected cells produce syncyntia-like, focal areas of infection. The syncytia will progress and detach leaving a hole in the monolayer. The focal areas of infection have been determined to be Type-B rotavirus by specific IFA staining of infected monolayers.

At time of harvest the remaining attached cells are removed by vigorous shaking, physical scrapping or successive freeze and thaws. At this point the cell debris can be concentrated (centrifugation or filtration) or used as is. The harvest is sonicated to break-up the debris and then the virus is pretreated with EDTA and subpassaged into fresh cell cultures as mentioned above. While sonication is the preferred method of disrupting infected cells, other methods such as homogenization or use of KCl may be employed. The newly inoculated cell cultures are again incubated for 1-7 days at 37-39° C and harvested as before. This inoculation, incubation and harvest of cell cultures has been continued for 50 times. The viral RNA genome has remained the same as the parent (virulent) strain through out passage in cell culture (Figure 1).

| Type-B rotavirus Passage Level in Ma-104's | Amount of Virus Present | Type-B Identification | |
|---|---|---|---|
| | | IFA¹ | GE² |
| AmB-1/Ma-1 | Approx 10⁵⁻⁶ particles/ml | + | + |
| AmB-1/Ma-12 | Approx 10³⁻⁴ particles/ml | | |
| AmB-1/Ma-25 | Approx 10³⁻⁴ particles/ml | + | + |
| AmB-1/Ma-40 | Approx 10⁴⁻⁵ particles/ml | | |
| AmB-1/Ma-50 | Approx 10⁶⁻⁷ particles/ml | + | + |

| | | | |
|---|---|---|---|
| 1. IFA: Indirect Immunofluorescent Staining | | | |
| 2. GE: RNA extraction PAGE evaluation; Rotaviral Fingerprint analysis. | | | |

The importance of the EDTA was shown by another experiment in which Type B rotavirus passaged 49 times in Ma-104's with EDTA (i.e., virus adapted to cell culture) was passaged in the absence of EDTA. By passage 8, there was little or no CPE. We could only make 3 passages of intestinal (unadapted) Type-B rotavirus in EDTA-free culture before loss of CPE.

### EXAMPLE 2

### PROPAGATION OF FOUR DIFFERENT PORCINE TYPE-B ROTAVIRUS ISOLATES IN THE MA-104 CELL LINE

Four porcine Type-B rotavirus isolates were determined to be different based on RNA genome profiles. Three of the isolates were from Iowa with two being isolated from diarrheic 4-5 week old weanling pigs and one isolated from a diarrheic 2-day old nursing pig. The fourth isolate was received from Indiana from 4-5 week old weanling pig with diarrhea. These isolates were propagated in Ma-104 cell cultures with technology described in Example-1.

It should be noted that two of the four isolates were a mixture of rotavirus Type A and B strains. After 2 passages in Ma-104 it was determined that the passaged virus was pure Type-B, i.e., the Type-A rotavirus had been eliminated.

The evaluation of the tenth cell culture passage material by RNA extraction PAGE assay demonstrated that all isolates had maintained their unique viral genome fingerprints for Type-B rotavirus. All tenth passage isolates were determined to be pure Type-B rotavirus by specific IFA staining of infected cell cultures.

### EXAMPLE 3

### PROPAGATION OF HUMAN TYPE-B ROTAVIRUS STRAIN(ADRV) IN MA-104 CELL LINE

Two fecal samples from different human volunteers (Subject #1 and #3) infected with the ADRV strain of Human Type-B rotavirus were evaluated in Ma-104's with techniques mentioned in Example 1. Three groups of roller tubes were inoculated with 4 tubes per group and 2 noninoculated cell control tubes. The first two groups were inoculated with Subject #1 feces, with the first set using unfilter sterilized inoculum and the second set using 0.2 micron filter sterilized inoculum. The final group was inoculated with Subject #3 unfiltered feces. After adsorption the inoculum was rinsed off three times, fresh virus propagation medium added and the tubes placed on a roller rack at 0.25 rpm at 37° C.

At 18 hours post-inoculation, typical Type-B cytopathic effect (CPE) was observed and by 24-hours post-inoculation the syncyntias had progressed and detached. It should be noted that even the filter sterilized group produced the typical syncyntia-like CPE formation, thus demonstrating it is not virus aggregates that infect the cell culture. All previous attempts to grow the Human Type-B, even initially in cell culture, have been unsuccessful.

### EXAMPLE 4

### DIAGNOSTIC KIT

The diagnostic assays of the present invention are not limited to any particular assay format. A fluoroimmunoassay, enzyme immunoassay, radioimmunoassay, particle (e.g., latex) agglutination assay, etc. might be employed. The assay may be in a competitive or a sandwich format and a labeled antigen may be employed instead of a labeled anti-antibody. For an EIA, the preferred labels are alkaline phosphatase and horseradish perodixase. For an RIA, the preferred label is I¹²⁵. Avidin-biotin linkages may be used to conjugate a labeled species (or a support) to an immunoreagent. Polyclonal or monoclonal antibodies raised against Type-B antigens may also be useful, after labeling or insolubilization, as diagnostic reagents.

Diagnostic kits are prepared from antigens or antibodies prepared according to the present invention by packaging the antigens or antibodies in suitable containers in suitable diluents.

Antiserum to the human Type-B rotavirus will cross-react with the Porcine Type-B rotavirus and porcine Type-B antiserum will cross-react with the Human Type-B rotavirus as evaluated by specific IFA and ELISA assays. In addition, published results indicate the Human Type-B rotavirus will cross-react (ELISA) with the bovine, rat and lamb Type-B rotaviruses (6,8,15,26). Two sources of Human Type-B antiserum were evaluated. The hyperimmune serum was kindly provided by Dr. Harry Greenburg, Veterans Medical Hospital, Stanford University. Serum was prepared in guinea pigs and rabbits. Leighton tubes of confluent Ma-104 cells were inoculated with the AmB-1/Ma-104 pass 25 as described in Example 1.

Slides were examined at 24-hours post-inoculation and typical CPE was observed. The slides were fixed in acetone and stained by Indirect Immunofluorescence using different dilutions of the human type-B antisera. Anti-rabbit and Anti-guinea pig IgG-FITC (Miles Laboratories) were used as conjugates. Specific Type-B fluorescence was seen at a 1/10 and 1/100 dilutions but not at 1/1000 dilution.

An antigen capture ELISA was evaluated using antiserum to Type-B rotavirus prepared in goats and guinea pigs. Type-B rotavirus bulk fluids propagated in Ma-104 cells as described in Example 1 were used as the immunizing agent for production of antiserum. The virus fluids were frozen and thawed three times and the cell debris removed by centrifugation at 10,000 x g for 30 min. The virus was further purified by ultracentrifugation (100,000 x g for 2 hours) through a sucrose gradient. Virus was collected and resuspended in sterile water. To remove any excess sucrose the virus was ultracentrifuged again as above and this time virus pellet was resuspended in sterile water.

Animals were hyperimmunized with virus adjuvanted in Freund's complete adjuvant, over a period of six weeks, with injections at one, three and five weeks.

Antiserum titers to the Type-B rotavirus were determined by ELISA block titrations against the purified Type-B rotavirus antigen described above using either anti-guinea pig or anti-goat IgG peroxidase conjugate and ABTS substrate.

The assay used for Type-B rotavirus detection was a sandwich ELISA where the microtiter plate was coated with a desired dilution of goat anti-Type-B rotavirus and incubate at 4°C overnight. The plates were washed with physiological saline and nonbound sites were blocked using 2% fetal bovine serum. The plates were washed with physiological saline and then inoculated with four fold dilutions (in physiological saline) of Type-B rotavirus bulk fluids generated as described in Example 1. As a control the last row of each plate was inoculated with four fold dilutions of Type-A rotavirus. After incubation for 1-hour at room temperature the plates were washed with physiological saline and Type-B guinea pig antiserum was added. After incubation for 1-hour at room temperature the plates were washed with physiological saline and rabbit anti-guinea pig horse radish peroxidase was added to all the wells. After a 1-hour incubation at room temperature, the plates were washed with physiological saline and ABTS substrate was added to each well. After incubation at room temperature in the dark for 1-hour the plates were read on an ELISA reader with a 410 nm filter. A positive reading was determined by samples giving a specific color reaction ≥ 0.1 optical density unit.

Cell culture passed porcine Type-B rotavirus has been found to be equivalent to virulent human Type-B rotavirus when used as an antigen source in an ELISA against the human Type-B serum. This demonstrates that the cell culture-adapted Type-B rotavirus will cross-react with the virulent Human Type-B rotavirus. Thus the adapted porcine Type-B described herein can be used in the preparation of antigen and antiserum for use in human and other mammalian diagnostic kits.

### EXAMPLE 5

### EVALUATION OF TISSUE CULTURE ADAPTED TYPE-B ROTAVIRUS AS MODIFIED LIVE VACCINE

An animal vaccine study was done to determine if the adapted Porcine Type-B rotavirus maintained its immunogenicity after 50 passages in cell culture with said technology. Six five day old CDCD pigs were used to evaluate different cell culture passage levels of the adapted Type-B porcine rotavirus. Two animals were nonvaccinated controls, two animals were vaccinated with type-B rotavirus Ma-104 passage 25 and two animals vaccinated with Type-B rotavirus Ma-104 passage 50. The titer of the AmB-1/Ma-25 vaccine was approximated to be 10⁵⁻⁶ TCID50/pig. The titer of the AmB-1/Ma-50 was approximated to be 10⁶⁻⁷ TCID50/pig. Intramuscular injections were adjuvanted with Freund's incomplete and animals were given only one oral and one intramuscular injection. At 3-weeks post-vaccination all animals were challenged with virulent Type-B rotavirus. Blood samples were collected at time of vaccination, time of challenge(3-weeks post-vaccination), and 3-weeks post-challenge. Serum antibody levels to Type-B rotavirus were determined by titration of the serum against infected cell culture slides. Briefly, serum dilutions were made and inoculated onto the infected slides, the slides were then washed and stained with rabbit anti-porcine IgG FITC conjugate. The slides were viewed for specific fluorescence and serum titers recorded as the reciprocal of the highest dilution that gave a positive reading. Data is presented in Table 1.

**Table 1:**

| **Serum Antibody Response to Cell Culture Adapted Type-B Porcine Rotavirus.** | | | | |
|---|---|---|---|---|
| Animal No. | Treatment | Type-B Rotavirus Serum Antibody Titers¹ | | |
| | | Prevac | Day of Challenge² | 3 weeks Post- Challenge |
| 1-1 | B Rota/Ma-25 | < 5 | < 5 | 400 |
| 1-2 | B Rota/Ma-25 | < 5 | < 5 | 100 |
| | | | | |
| 2-1 | B Rota/Ma-50 | < 5 | 100 | 400 |
| 2-2 | B Rota/Ma-50 | < 5 | 100 | 400 |
| | | | | |
| 3-1 | Nonvaccinated | < 5 | < 5 | 80 |
| 3-2 | Nonvaccinated | < 5 | < 5 | 40 |

| | | | | |
|---|---|---|---|---|
| 1 Titers: Titers are expressed as the reciprocal of the highest dilution that elicited a positive IFA titer. | | | | |
| 2 Three weeks post-vaccination. | | | | |

Although the animals that received the lower passage of Type-B rotavirus did not demonstrate a seroconversion post-vaccination it is apparent they were at least initially primed as the level of Type-B antibodies post-challenge is the same for both the 50th and 25th Type B rotavirus passage levels. Antibody levels for both vaccinate groups were at least 5 times greater than the nonvaccinated challenge control antibody levels. The relatively low titers in animal 1-2 and 1-1 may have been due to the fact the AmB-1/Ma-25 vaccinates received approximately 10x less virus than the AmB-1/Ma-50 vaccinates.

Animals were observed for clinical signs of rotavirus infection twice daily. No animal demonstrated any clinical signs post-vaccination, thus demonstrating the safety of the Type-B tissue culture passaged material. Data suggests the virus has been modified to allow an immune response without virulence factors associated with disease. In addition the clinical signs post-challenge show the vaccinates to have a lower morbidity incidence then nonvaccinated control animals (Table 2). Morbidity Incidence and Duration (MID) is defined as the number of days the pigs show diarrhea/total number of pig days (7-day post-challenge period).

**Table-2:**

| **Clinical Signs Post-Challenge** | | |
|---|---|---|
| Group | MID | % Reduction Compared to Nonvaccinated |
| Nonvaccinated | 12/14 (86%) | |
| Type-B/Ma-25 | 9/14 (64%) | 26 % |
| Type-B/Ma-50 | 0/14 (0%) | 100 % |

These data clearly demonstrate that the adaptation of Type-B rotavirus to cell culture by the methods taught herein is not deleterious to its antigenicity and that virus propagated in cell culture by our technique may lead to effective vaccines (MLV or Killed) against virulent Type-B rotavirus infections. Passage in cell culture with said technique will diminish or eliminate Type-B rotavirus virulence, without altering its immunogenicity.

### EXAMPLE 6

### WEANING OF VIRUS FROM EDTA

We have found that after repeated passages in which virus fluids are pretreated or supplemented with EDTA, the Rota-B may be weaned from the EDTA without lasting impairment of growth. In the experiment below, we considered the effect of weaning the virus from EDTA at the 22nd passage.

| Passage | EDTA-Retained | EDTA-Weaned |
|---|---|---|
| 22 | 10³ TCID ₅₀/ml | 10¹ TCID ₅₀/ml |
| 35 | 10⁴ | 10³ |
| 50 | 10⁴ | 10⁵⁻⁶ |

Passages were about two days apart.

It is apparent from this data that by the 50th passage, the virus has fully recovered from the shock of weaning from EDTA.

The foregoing description of the specific embodiments will so fully reveal the general nature of the invention that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation.

### REFERENCES

1. Bellinzoni, N., Mattion, L., Vallejos, J., La Torre, E., Scodeller, A. 1987. Atypical Rotavirus in Chickens in Argentina. Res. Vet Science, 43: 130-131.
2. Benfield, D.A., Stotz, Ivan., Moore, R. and McAdaragh, John P.1982. Shedding of Rotavirus in Feces of Sows Before and After Farrowing. Clin. Microbiol., 16: 186-190.
3. Bohl, E.H., Kohler, E.M., Saif, L.J., Cross, R.F., Agnes, A.G. and Theil, K.W. 1978. Rotavirus as a Cause of Diarrhea in Pigs. J. Am. Vet. Med. Assoc., 172: 458-463.
4. Bridger, J.C. 1988. Porcine Rotaviruses and their Role in Disease. Pig News and Information, 9: 23-26.
5. Bridger, J.C., Brown, J.F., 1985. Prevalence of Antibody to Typical and Atypical Rotaviruses in Pigs. Vet. Rec., 116:50.
6. Brown, D. W., Beards, G. M., Guang-Mu, C., Flewett, T. H., 1987. Prevalence of Antibody to Group B (Atypical) Rotavirus in Humans and Animals. J. Clin. Micro., 25: 316-319.
7. Chasey, D., Banks, J., 1984. The Commonest Rotaviruses from Neonatal Lamb Diarrhea in England and Wales have Atypical Electropherotypes. Vet Rec., 115: 326-327.
8. Eiden, J., Vonderfecht, S., Yolken, R. H. 1985. Evidence that a Novel Rotavirus-Like Agent of Rats can cause Gastroenteritis in Man. Lancet ii: 8-11.
9. Estes, M., K., Graham, D. Y., Smith, E. M., Gerba, C. P. 1979. Rotavirus Stability and Inactivation. J. Gen. Virol. 43:403.
10. Fitzgerald, G.R. Barker, T. Welter, M.W. and Welter, C.J. 1988. Diarrhea in Young Pigs: Comparing the Incidence of the Five Most Common Infectious Agents. Vet. Med., 83:80-86.
11. Fitzgerald, G.R., Welter, M.W. and Welter, C.J. 1986. Evaluating the Performance of a Porcine Rotavirus Vaccine. Vet. Med., 81:188-192.
12. Fitzgerald, G.R., Welter, M.W. and Welter, C.J. 1986. Effect of Porcine Rotavirus Vaccination on Postweaning Weight Gains in Baby Pigs. Modern Vet. Pract., 67: 609-610.
13. Hung, T., Chen, G., Wang, C., Yao, H., Fang, Z., Chao, T., Chou, Z., Ye, W., Chang, X., Den, S., Liong, X., Chang, W. 1984. Waterborn Outbreak of Rotavirus Diarrhea in Adults in China Caused by a Novel Rotavirus. Lancet ii:1139-1142.
14. Kapikian, A. Z., Flores, J., Hoshino, Y., Midthun, K., Gorziglia, M., Green, K, Y., Chanock, R. M., Potash, L., Sears, S, D., Clements, M, L., Halsey, N, A., Black, R, E., Perez-Schael, I. 1989. Prospects for Development of a Rotavirus Vaccine Against Rotavirus Diarrhea in Infants and Young Children. Rev. of Infect. Dis. Vol II, Supplement 3:S539-S546.
15. Nakata, S., Estes, M. K., Graham, D. Y., Loosle, R., Too, H., Shusheng, W., Saif, L. J., Melnick, J. L. 1986. Antigenic Characterization and ELISA Detection of Adult Diarrhea Rotavirus. J. Infect. Disease., 154(3):448-454.
16. Saif, L, J., Terrett, L, A., Miller, K, L., Cross, R, F. 1988. Serial Propagation of Porcine Group C Rotavirus(Pararotavirus) in a Continuous Cell Line and Characterization of the Passaged Virus. J. Clin. Micro., 26(7):1277-1282.
17. Snodgrass, D. R., Herring, A. J., Campbell, I., Inglis, J. M., Hargreares, F. D. 1984. Atypical Rotaviruses from Calves, Piglets, Lambs and Man. J. Gen. Vir., 65:909-914.
18. Theil, K. W., McCloskey, C. M., Saif, L. J., Redman, D. R., Bohl, E. H., Hancock, D. D., Kohler, E. M., Moorhead, P. D. 1981. Rapid, simple method of Preparing Rotaviral Double-Stranded Ribonucleic Acid for Analysis by Polyacrylamide Gel electrophoresis. J. Clin. Microbiol. 14:273-280.
19. Theil, K. W., Saif, L. J. 1985. In Vitro Detection of Porcine Rotavirus-Like Virus (Group B Rotavirus) and It's Antibody. J. Clin. Micro., 21(5):844-846.
20. Theil, K.W., Saif, L.J., Moorhead, P.D., Whitmoyer, R.E. 1985. Porcine Rotavirus-Like Virus (Group B Rotavirus): Characterization and Pathogenicity for Gnotobiotic Pigs. J. Clin. Micr., 21(3):340-345.
21. Vonderfecht, S. L., Eiden, J. J., Torres, A., Miskuff, R. L., Mebus, C. A., Yolken, R. H. 1986. Identification of a Bovine Enteric Syncytial Virus as a Nongroup A Rotavirus. American J. Vet Res., 47(9): 1913-1918.
22. Vonderfecht, S. L., Huber, A. C., Eiden, J., Mader, L. C., Yolken, R. H. 1984. Infectious Diarrhea of Infant Rats Produced by a Rotavirus-Like Agent. J. Virol. 52: 94-98.
23. Welter, M.W., Fitzgerald, G.R., and Welter, C.J. 1986. A Combination Porcine Rotavirus Vaccine Against Two Major Type-A Serotypes. Agri. Practice Swine Immunology. 7:59-62.
24. Welter, M. W., Welter, C. J. 1990. Evaluation of Killed and Modified Live Porcine Rotavirus Vaccines in Cesarean Derived Colostrum Deprived Pigs, Vet Micro. 22: 179-186.
25. Woode, G.N., Bridger, J.C., Hall, G.A., Jones, J.M. and Jackson, G. 1976. The Isolation of Reovirus-Like Agents (Rotaviruses) from Acute Gastroenteritis of Piglets. J. Med. Microbiol., 9:203-209.
26. Yolken, R., Wee, S., Eiden, J., Kinney, J., Vonderfecht, S. 1988. Identification of a Group Reactive Epitope of Group B Rotaviruses Recognized by Monoclonal Antibody and Application to the Development of a Sensitive Immunoassay for Viral Characterization. J. Clin. Micr., 26(9):1853-1858.
27. U.S. PATENT: 3,838,004, Mebus and Twiehaus, 9-24-74, Calf Diarrhea Virus Vaccine and Processes.
28. U.S. Patent: 3,839,556, Mebus and Twiehaus, 10-1-74, Calf Diarrhea Virus Vaccine and Processes.
29. U.S. Patent: 3,869,547, Mebus et al., 3-4-75, Calf Diarrhea Virus Vaccine and Processes.
30. U.S. Patent: 4,751,080, Wyatt et al., 6-14-88, Vaccine Against Rotavirus Diseases.
31. Pedley, S., Bridger, J.C., Brown, J.F., McCrae, M.A. 1983. Molecular Characterization of Rotaviruses with Distinct Group Antigens. J. Gen. Virol., 64: 2093-2101.

## Claims

1. A method of adapting a Type B rotavirus to continuous growth in cell culture comprising serial passaging a Type B rotavirus in cells capable of supporting the growth of the rotavirus, said cells being provided with a medium which supports the growth of said cells and which is essentially free of proteolytic enzymes to which said Type B rotavirus is sensitive, the virus being exposed to a chelating agent prior to or during said passaging and remaining infectious for said cells.

2. The method of claim 1 wherein the medium contains less than 0.6 mg/ml Ca⁺⁺ and less than 0.4 mg/ml Mg⁺⁺.

3. The method of claims 1 or 2 wherein the chelating agent is EDTA and EGTA.

4. The method of any of claims 1-3 wherein the medium further comprises a detergent such as sodium dodecyl sulfate.

5. The method of any of claims 1-4 wherein the cells are selected from the group consisting of bovine, porcine and monkey cells.

6. The method of any of claims 1-5 in which the cells are selected from the group consisting of bovine turbinate, embryonic bovine kidney, swine testicular, primary pig kidney, Vero, Bsc, CV-1, embryonic bovine lung, and embryonic rhesus monkey cells.

7. The method of any of claims 1-6 in which the cells are Ma-104 cells.

8. The method of claim 1, wherein the rotavirus is passaged in the medium for more than three passages, preferably for at least 9 passages.

9. A method of propagating a Type B rotavirus in cell culture which comprises adapting the rotavirus to cell culture by the method of any of claims 1-8 and then weaning the virus from the chelating agent.

10. The method of claim 9, wherein the rotavirus is weaned from the chelating agent after at least about twenty passages in which the virus is exposed to the chelating agent.

11. An attenuated Type B rotavirus vaccine comprising Type B rotavirus obtained by growing the virus by the method according to any of claims 1-10.

12. Use of a Type B rotavirus propagated in cell culture according to the method of any of claims 1-10 for the preparation of a vaccine for immunizing or treating a human or animal for an infection caused by a Type B rotavirus.

13. Use of an immunogenic composition comprising one or more Type B rotavirus antigens obtained by a process comprising adapting a Type B rotavirus to cell culture according to the method of any of claims 1-10 and lysing the host cells, and a pharmaceutically acceptable carrier, said composition being capable of raising antibodies in an immunocompetent subject, said antibodies being immunologically cross-reactive with Type B rotavirus, for the preparation of an immunogenic composition for immunizing a human or animal.

14. The use according to claim 12 or 13 in which the propagated or adapted rotavirus is a human rotavirus, and a human is immunized or treated.

15. The use according to claim 12 or 13 in which the propagated or adapted rotavirus is a porcine rotavirus, and a human is immunized or treated.

16. The use according to claim 12 or 13 in which the propagated or adapted rotavirus is a porcine rotavirus, and the animal immunized or treated is a pig.

17. The use according to claim 12 or 13 in which the propagated or adapted rotavirus is a human rotavirus, and the animal immunized or treated is a pig.

## Patentansprüche

1. Verfahren zum Adaptieren eines Rotavirus-Typ-B an kontinuierliches Wachstum in Zellkultur, umfassend aufeinanderfolgendes Passagieren eines Rotavirus-Typ-B in Zellen, die das Wachstum des Rotavirus unterstützen können, wobei die Zellen mit einem Medium versorgt werden, das das Wachstum der Zellen unterstützt und das im wesentlichen frei von proteolytischen Enzymen ist, für die das Rotavirus-Typ-B sensitiv ist, wobei der Virus vor oder während des Passagierens einem komplexbildenden Mittel ausgesetzt wird und für diese Zellen infektiös bleibt.

2. Verfahren nach Anspruch 1, wobei das Medium weniger als 0,6 mg/ml Ca⁺⁺ und weniger als 0,4 mg/ml Mg⁺⁺ enthält.

3. Verfahren nach Anspruch 1 oder 2, wobei das komplexbildende Mittel EDTA und EGTA ist.

4. Verfahren nach einem der Ansprüche 1 bis 3, wobei das Medium weiterhin ein Detergens wie Natriumdodecylsulfat enthält.

5. Verfahren nach einem der Ansprüche 1 bis 4, wobei die Zellen ausgewählt sind aus Rinder-, Schwein- und Affen-Zellen.

6. Verfahren nach einem der Ansprüche 1 bis 5, bei dem die Zellen ausgewählt sind aus Rindernasenmuschel-, embryonalen Rindernieren-, Schweinehoden-, primären Schweinenieren-, Vero-, Bsc-, CV-1-, embryonalen Rinderlungen-, und embryonalen Rhesus-Affen-Zellen.

7. Verfahren nach einem der Ansprüche 1 bis 6, bei dem die Zellen Ma-104-Zellen sind.

8. Verfahren nach Anspruch 1, wobei das Rotavirus in dem Medium für mehr als drei Passagen, vorzugsweise für mindestens neun Passagen, passagiert wird.

9. Verfahren zum Vermehren eines Rotavirus-Typ-B in Zellkultur, das das Adaptieren des Rotavirus an die Zellkultur durch das Verfahren nach einem der Ansprüche 1 bis 8 und dann das Entwöhnen des Virus von dem komplexbildenden Mittel umfaßt.

10. Verfahren nach Anspruch 9, wobei das Rotavirus nach mindestens ungefähr 20 Passagen, bei denen das Virus dem komplexbildenden Mittel ausgesetzt ist, von dem komplexbildenden Mittel entwöhnt wird.

11. Attenuierte Rotavirus-Typ-B-Vakzine, enthaltend Rotavirus-Typ-B, der durch Wachstum des Virus nach dem Verfahren gemäß einem der Ansprüche 1 bis 10 erhalten wurde.

12. Verwendung eines Rotavirus-Typ-B, der gemäß dem Verfahren nach einem der Ansprüche 1 bis 10 in Zellkultur vermehrt wurde, zur Herstellung einer Vakzine zur Immunisierung oder Behandlung eines Menschen oder eines Tieres gegen eine Infektion, die durch einen Rotavirus-Typ-B verursacht wird.

13. Verwendung einer immunogenen Zusammensetzung, enthaltend ein oder mehrere Rotavirus-Typ-B-Antigene, die durch ein Verfahren erhalten wurden, das das Adaptieren eines Rotavirus-Typ-B an die Zellkultur gemäß dem Verfahren nach einem der Ansprüche 1 bis 10 und das Lysieren der Wirtszellen umfaßt, und einen pharmazeutisch verträglichen Träger, wobei die Zusammensetzung Antikörper in einem immunkompetenten Subjekt hervorrufen kann, wobei die Antikörper auf immunologische Weise mit Rotavirus-Typ-B kreuzreaktiv sind, zur Herstellung einer immunogenen Zusammensetzung zur Immunisierung eines Menschen oder Tieres.

14. Verwendung nach Anspruch 12 oder 13, bei der das vermehrte oder adaptierte Rotavirus ein menschliches Rotavirus ist und ein Mensch immunisiert oder behandelt wird.

15. Verwendung nach Anspruch 12 oder 13, bei der das vermehrte oder adaptierte Rotavirus ein Schweine-Rotavirus ist und ein Mensch immunisiert oder behandelt wird.

16. Verwendung nach Anspruch 12 oder 13, bei der das vermehrte oder adaptierte Rotavirus ein Schweine-Rotavirus ist und das immunisierte oder behandelte Tier ein Schwein ist.

17. Verwendung nach Anspruch 12 oder 13, bei der das vermehrte oder adaptierte Rotavirus ein menschliches Rotavirus ist und das immunisierte oder behandelte Tier ein Schwein ist.

## Revendications

1. Procédé d'adaptation d'un rotavirus de Type B à la croissance continue dans une culture cellulaire, dans lequel on fait passer successivement un rotavirus de Type B dans des cellules capables d'entretenir la croissance du rotavirus, lesdites cellules étant munies d'un milieu qui entretient la croissance desdites cellules et qui est essentiellement dépourvu d'enzymes protéolytiques auxquelles ledit rotavirus du Type B est sensible, le virus étant exposé à un agent chélatant avant ou pendant ledit passage et restant infectieux pour lesdites cellules.

2. Procédé de la revendication 1 dans lequel le milieu contient moins de 0,6 mg/ml de Ca⁺⁺ et moins de 0,4 mg/ml de Mg⁺⁺.

3. Procédé des revendications 1 ou 2 dans lequel l'agent chélatant est l'EDTA et l'EGTA.

4. Procédé de l'une quelconque des revendications 1-3 dans lequel le milieu comprend en outre un détergent comme le dodécyl-sulfate de sodium.

5. Procédé de l'une quelconque des revendications 1-4 dans lequel les cellules sont choisies dans le groupe constitué par les cellules de boeuf, de porc et de singe.

6. Procédé de l'une quelconque des revendications 1-5 dans lequel les cellules sont choisies dans le groupe constitué par les cellules de cornet de boeuf, les cellules embryonnaires de rein bovin, les cellules de testicule de porc, les cellules primaires de rein de porc, les cellules Vero, Bsc, CV-1, les cellules embryonnaires de poumon bovin, et les cellules embryonnaires de singes rhésus.

7. Procédé de l'une quelconque des revendications 1-6 dans lequel les cellules sont des cellules Ma-104.

8. Procédé de la revendication 1, dans lequel on fait passer le rotavirus dans le milieu plus de trois fois, et de préférence pour au moins 9 passages.

9. Procédé de propagation d'un rotavirus de Type B dans une culture cellulaire dans lequel on adapte le rotavirus à une culture cellulaire par le procédé de l'une quelconque des revendications 1-8 puis on sèvre le virus de l'agent chélatant.

10. Procédé de la revendication 9, dans lequel on sèvre le rotavirus de l'agent chélatant après au moins environ vingt passages dans lesquels le virus est exposé à l'agent chélatant.

11. Vaccin à rotavirus de Type B atténué comprenant un rotavirus de Type B obtenu en cultivant le virus par le procédé selon l'une quelconque des revendications 1-10.

12. Utilisation d'un rotavirus de Type B propagé dans une culture cellulaire selon le procédé de l'une quelconque des revendications 1-10 pour la préparation d'un vaccin pour immuniser ou traiter un être humain ou un animal pour une infection provoquée par un rotavirus de Type B.

13. Utilisation d'une composition immunogène comprenant un ou plusieurs antigènes de rotavirus de Type B obtenus par un procédé dans lequel on adapte un rotavirus de Type B à une culture cellulaire selon le procédé de l'une quelconque des revendications 1-10 et on lyse les cellules hôtes, et un support pharmaceutiquement acceptable, ladite composition étant capable de susciter des anticorps chez un sujet immunocompétent, lesdits anticorps présentant une réaction immunologique croisée avec un rotavirus de Type B, pour la préparation d'une composition immunogène pour immuniser un être humain ou un animal.

14. Utilisation selon la revendication 12 ou 13 dans laquelle le rotavirus propagé ou adapté est un rotavirus humain, et on immunise ou on traite un être humain.

15. Utilisation selon la revendication 12 ou 13 dans laquelle le rotavirus propagé ou adapté est un rotavirus de porc, et on immunise ou on traite un être humain.

16. Utilisation selon la revendication 12 ou 13 dans laquelle le rotavirus propagé ou adapté est un rotavirus de porc, et l'animal immunisé ou traité est un porc.

17. Utilisation selon la revendication 12 ou 13, dans laquelle le rotavirus propagé ou adapté est un rotavirus humain, et l'animal immunisé ou traité est un porc.
